# EUROPÄISCHE PATENTSCHRIFT

(11) **EP 1 294 672 B1**
(45) Veröffentlichungstag und Bekanntmachung des Hinweises auf die Patenterteilung: **25.02.2004**
(21) Anmeldenummer: 01945319.0
(22) Anmeldetag: 27.06.2001
(51) Int. Cl.: C07C 51/235, C07C 51/215, C07C 57/05, C07C 57/055, C07C 45/50, C07C 45/35, C07C 47/02, C07C 47/22

(54) **VERFAHREN ZUR HERSTELLUNG VON HYDROFORMYLIERUNGSPRODUKTEN DES PROPYLENS UND VON ACRYLSÄURE UND/ODER ACROLEIN**
METHOD FOR PRODUCING PROPYLENE HYDROFORMYLATION PRODUCTS AND ACRYLIC ACID AND/OR ACROLEIN
PROCEDE DE PRODUCTION DE PRODUITS D'HYDROFORMYLATION DU PROPYLENE ET D'ACIDE ACRYLIQUE ET / OU D'ACROLEINE

(30) Priorität: 28.06.2000 DE 10031518
(43) Veröffentlichungstag der Anmeldung: 26.03.2003
(73) Patentinhaber: BASF AKTIENGESELLSCHAFT, 67056 Ludwigshafen (DE)
(72) Erfinder: KROKOSZINSKI, Roland, 67273 Weisenheim a. Berg (DE); HAMMON, Ulrich, 68165 Mannheim (DE); TODD, Kevin, Lake Jackson, TX 77566 (US)
(74) Vertreter: Kinzebach, Werner, Dr.
(86) Internationale Anmeldenummer: PCT/EP2001/007337
(87) Internationale Veröffentlichungsnummer: WO 2002/000587

(56) Entgegenhaltungen:
- EP-A- 0 648 730
- US-A- 4 259 211
- US-A- 4 298 763

## Beschreibung

Die vorliegende Erfindung betrifft ein Verfahren zur Herstellung von Hydroformylierungsprodukten des Propylens und von Acrylsäure und/oder Acrolein.

Die Hydroformylierung von Propylen ist ein wichtiges großtechnisches Verfahren zur Herstellung von Hydroformylierungsprodukten des Propylens, d. h. Butyraldehyd und/oder Butanol.

Die Hydroformylierung von Propylen erfolgt großtechnisch in einem kontinuierlichen Verfahren, bei dem Propylen zusammen mit Kohlenmonoxid und Wasserstoff in einen Hydroformylierungsreaktor eingespeist und in Gegenwart eines Hydroformylierungskatalysators umgesetzt wird. Der Reaktionsaustrag enthält neben den Hydroformylierungsprodukten von Propylen in der Regel bedeutende Mengen an nicht umgesetztem Propylen, das abgetrennt und in der Regel zusammen mit frischem Kohlenmonoxid und Wasserstoff wieder dem Hydroformylierungsreaktor zugeführt wird. Mit dem zurückgeführten Propylen wird allerdings auch das als Verunreinigung im zugeführten Propylen enthaltene oder durch Nebenreaktionen im Hydroformylierungsreaktor gebildete Propan, das der Hydroformylierung nicht zugänglich ist, in den Reaktor zurückgeführt. Um zu verhindern, dass die Propankonzentration im Hydroformylierungsreaktor kontinuierlich ansteigt und Werte erreicht, bei denen die Hydroformylierungsreaktion zum Erliegen kommt, muss laufend ein Teilstrom des zurückgeführten Propylen enthaltenden Stroms aus dem Verfahren ausgeleitet werden, um das mit dem Propylenzulauf eingeführte bzw. durch Nebenreaktion gebildete Propan aus dem System zu entfernen.

Mit dem Ausleitstrom wird neben Propan auch nicht umgesetztes Propylen aus dem System entfernt, das somit für die weitere Umsetzung verloren ist. Um diese Verluste gering zu halten, wird im Allgemeinen ein Propylenzulauf hoher Reinheit eingesetzt. Zur Hydroformylierung wird üblicherweise ein Propylenzulauf einer Reinheit von etwa 99,5 % eingesetzt, das bisweilen als "Polymer Grade Propylen" bezeichnet wird, wobei der Rest im Wesentlichen aus Propan besteht. Ein derartiger Propylenzulauf hoher Reinheit ist allerdings nur aufwendig zu erhalten und wird demzufolge zu deutlich höheren Preisen auf dem Markt angeboten als Propylene niedrigerer Reinheit. So ist beispielsweise das sogenannte "Chemical Grade Propylen", das etwa 3 bis 7 % Propan enthält, deutlich billiger als das angesprochene "Polymer Grade Propylen".

Ein Propylenzulauf mit einem höheren Anteil an Propan kann in einem industriellen Hydroformylierungsverfahren aus den genannten Gründen nicht ohne Weiteres eingesetzt werden. Um zu verhindern, dass die Propankonzentration im Hydroformylierungsreaktor derart hohe Werte erreicht, dass die Hydroformylierung zum Erliegen kommt, müsste der Ausleitstrom unter gleichzeitigem Verlust von nicht umgesetztem Propylen derart hoch gewählt werden, dass die Ersparnis des billigeren Einsatzstoffs zunichte gemacht würde.

Es ist bereits vorgeschlagen worden, den vom Hydroformylierungsprodukt abgetrennten Strom in eine Propylen-angereicherte und eine Propylen-abgereicherte Fraktion zu trennen und nur die Propylen-angereicherte Fraktion in die Reaktionszone zurückzuführen. Die Trennung von Propylen und Propan ist allerdings aufgrund ihrer ähnlichen physikalischen Eigenschaften aufwendig. So offenbart die EP-A-0648730 ein Verfahren zur Herstellung eines aus Propylen gewonnenen Oxoprodukts, bei dem ein aus dem Produktstrom einer Propylen-Hydroformylierung abgetrennter Gasstrom einer selektiven Adsorption des Propylens an ein Adsorptionsmittel und anschließende Desorption unterworfen wird. Die alternierenden Adsorptions- und Desorptionscyclen erfordern periodische Druckund/oder Temperaturwechsel. Die dazu benötigten Apparaturen sind aufwendig und störanfällig.

Acrylsäure ist eine bedeutende Grundchemikalie. Aufgrund ihrer reaktionsfähigen Doppelbindung eignet sie sich insbesondere als Monomeres zur Herstellung von Polymerisaten. Von der hergestellten Menge an Acrylsäure wird der größere Teil vor der Polymerisation - zu z. B. Klebstoffen, Dispersionen oder Lacken - verestert. Ein Teil der hergestellten Acrylsäure wird direkt - zu z. B. "Superabsorbern" - polymerisiert. Es ist bekannt, dass Acrylsäure durch heterogen katalysierte Gasphasenoxidation von Propylen mit molekularem Sauerstoff an festen Katalysatoren hergestellt werden kann, vergleiche z. B. DE-A-1962431, DE-A-2943707, DE-C-1205502, DE-A-19508558, EP-A-0257565, EP-A-0253409, DE-A-2251364, EP-A-0117146, GB-B-150986 und EP-A-0293224.

Eine Kopplung der Herstellung von Acrylsäure und/oder Acrolein und der Herstellung von Hydroformylierungsprodukten des Propylens ist im Stand der Technik nicht beschrieben oder angeregt.

Der vorliegenden Erfindung liegt die Aufgabe zugrunde, ein Verfahren zur Herstellung von Hydroformylierungsprodukten des Propylens anzugeben, das den Einsatz von Propylenzuläufen mit einer Beimischung von Propan erlaubt, und eine möglichst vollständige stoffliche Nutzung des zugeführten Propylens und/oder Propans gestattet.

Überraschenderweise wurde nun gefunden, dass ein vom Reaktionsaustrag der Hydroformylierung von Propylen abgetrennter, im Wesentlichen aus nicht umgesetztem Propylen und Propan bestehender Strom als Einsatzmaterial zur Herstellung von Acrylsäure und/oder Acrolein durch katalytische Gasphasenoxidation geeignet ist.

Gegenstand der Erfindung ist daher ein Verfahren zur Herstellung von Hydroformylierungsprodukten des Propylens und von Acrylsäure, bei dem man
a) in eine Reaktionszone einen Propylen-haltigen Zulauf sowie Kohlenmonoxid und Wasserstoff einspeist und in Gegenwart eines Hydroformylierungskatalysators zu Hydroformylierungsprodukten des Propens umsetzt,
b) vom Austrag aus der Reaktionszone einen im Wesentlichen aus nicht umgesetztem Propylen und Propan bestehenden Strom abtrennt,
c) den im Wesentlichen aus Propylen und Propan bestehenden Strom in Gegenwart von molekularem Sauerstoff einer katalytischen Gasphasenoxidation zu Acrylsäure und/oder Acrolein unterwirft.

Die Erfinder haben gefunden, dass die spezifischen Verunreinigungen, die im vom Reaktionsaustrag der Hydroformylierung abgetrennten Propylen/Propan-Strom enthalten sein können, wie Kohlenmonoxid, Wasserstoff, Butyraldehyd, Butanol usw., die Aktivität, Selektivität und Standzeit der bei der Gasphasenoxidation von Propylen zu Acrylsäure und/oder Acrolein eingesetzten heterogenen Katalysatoren nicht beeinträchtigen und sich auch bei der Aufarbeitung der dabei erhaltenen Reaktionsprodukte nicht störend auswirken.

Geeignete druckfeste Reaktionsapparaturen zur Durchführung der Hydroformylierung sind dem Fachmann bekannt. Dazu zählen die allgemein üblichen Reaktoren für Gas-Flüssig-Reaktionen, wie z. B. Rührkessel, Gasumlaufreaktoren, Blasensäulen usw., die gegebenenfalls durch Einbauten weiter unterteilt sein können.

Der als Ausgangsmaterial für das erfindungsgemäße Verfahren geeignete Propylenzulauf kann neben Propylen eine Beimischung von Propan enthalten. Er enthält z. B. 0,5 bis 40 Gew.-%, vorzugsweise 2 bis 30 Gew.-.%, insbesondere 3 bis 10 Gew.-%, Propan. Ein bevorzugtes Beispiel ist "Chemical Grade Propylen", das 3 bis 10 Gew.-% Propan enthält. Es wird z. B. durch Umsetzung von Naphtha oder Erdgas im Steamcracker und anschließende destillative Aufarbeitung erhalten. Ein weiteres Beispiel eines geeigneten Propylenzulaufs ist auch das sogenannte "Refinery Grade Propylen", das Propangehalte von 20 bis 30 % aufweist.

Kohlenmonoxid und Wasserstoff werden üblicherweise in Form eines Gemischs, dem sogenannten Synthesegas, eingesetzt. Die Zusammensetzung des im erfindungsgemäßen Verfahren eingesetzten Synthesegases kann in weiten Bereichen variieren. Das molare Verhältnis von Kohlenmonoxid und wasserstoff beträgt in der Regel 5:1 bis 1:5, vorzugsweis 2:1 bis 1:2, insbesondere etwa 45:55.

Die Temperatur bei der Hydroformylierungsreaktion liegt im Allgemeinen in einem Bereich von 50 bis 200 °C, vorzugsweise etwa 60 bis 190 °C, insbesondere etwa 90 bis 190 °C. Die Umsetzung wird vorzugsweise bei einem Druck im Bereich von etwa 10 bis 700 bar, vorzugsweise 15 bis 200 bar, insbesondere 15 bis 60 bar durchgeführt. Der Reaktionsdruck kann in Abhängigkeit von der Aktivität des eingesetzten Hydroformylierungskatalysators variiert werden.

Geeignete Hydroformylierungskatalysatoren sind die üblichen, dem Fachmann bekannten Übergangsmetallverbindungen und -komplexe, die sowohl mit als auch ohne Cokatalysatoren eingesetzt werden können. Bevorzugt handelt es sich bei dem Übergangsmetall um ein Metall der VIII. Nebengruppe des Periodensystems und insbesondere um Co, Ru, Rh, Pd, Pt, Os oder Ir, insbesondere um Rh, Co, Ir oder Ru. Besonders bevorzugte Hydroformylierungskatalysatoren zur Hydroformylierung von Propylen sind phosphorhaltige Rhodiumkatalysatoren, wie RhH(CO)₂(PPh₃)₂ oder RhH(CO)(PPh₃)₃. Geeignete Hydroformylierungskatalysatoren werden z. B. in Beller et al., Journal of Molecular Catalysis A, 104 (1995), S. 17-85 beschrieben, worauf im vollen Umfang Bezug genommen wird.

zur Gewinnung eines Propylen und Propan enthaltenden Stroms bietet der Stand der Technik eine Vielzahl technischer Verfahren an, deren Anwendung für den Erfolg des erfindungsgemäßen Verfahrens in der Regel nicht kritisch ist, solange bestimmte Vorgaben erfüllt werden. So kann der Austrag aus der Reaktionszone einer ein- oder mehrstufigen Trennoperation unterzogen werden, wobei zumindest ein die Hauptmenge des Hydroformylierungsproduktes, d. h. Butyraldehyd und/oder Butanol, enthaltender Strom und ein im wesentlichen aus nicht umgesetztem Propylen und Propan bestehender Strom erhalten werden. In Abhängigkeit von Faktoren, wie der Art des Austragsverfahrens, Reinheit des eingesetzten Synthesegases usw., werden gegebenenfalls weitere Ströme erhalten, wie Synthesegas-haltige Abgase, hochsiedende Nebenprodukte der Hydroformylierung und/oder Hydroformylierungskatalysator enthaltende Ströme, die - gegebenenfalls nach Aufarbeitung - ganz oder teilweise in die Reaktionszone zurückgeführt oder aus dem Verfahren ausgeschleust werden. Vom Austrag aus der Reaktionszone können beispielsweise zuerst das Hydroformylierungsprodukt und gegebenenfalls höher als das Hydroformylierungsprodukt siedende Anteile abgetrennt werden. Anschließend kann ein Gemisch von nicht umgesetztem Propylen und Propan - z. B. durch Abkühlung - auskondensiert werden.

Zweckmäßigerweise erhält man den im Wesentlichen aus nicht umgesetztem Propylen und Propan bestehenden Strom jedoch dadurch, dass man vom Austrag aus der Reaktionszone z.B. in einem Gas-Flüssigkeits-Abscheider, gegebenenfalls nach vorheriger Entspannung des Austrags auf einen niedrigeren Druck als den im Reaktor vorherrschenden, zuerst ein rohes Hydroformylierungsprodukt abtrennt, welches nicht umgesetztes Propylen und Propan gelöst enthält, und das rohe Hydroformylierungsprodukt dann einem Entgasungsschritt unterzieht, bei dem ein Strom anfällt, der im Wesentlichen aus nicht umgesetztem Propylen und Propan besteht. Der Rest des vom rohen Hydroformylierungsprodukt abgetrennten Reaktionsaustrags, der im allgemeinen nicht-umgesetztes Synthesegas, Inertgas (z.B. Methan, N₂, CO₂) sowie einen Teil des im Reaktionsaustrag enthaltenen nicht umgesetzten Propylens und des Propans enthält, wird in der Regel ganz oder teilweise in die Reaktionszone zurückgeführt.

Zur Entgasung des gelösten Propylens und Propans kann das rohe Hydroformylierungsprodukt dann gegebenenfalls weiter entspannt, erwärmt und/oder mit einem Strippgas, z.B. Synthesegas, behandelt werden. Zweckmäßigerweise führt man die Entgasung in einer Kolonne durch, wobei das rohe Hydroformylierungsprodukt im Bereich der Kolonnenmitte eingespeist und am Kolonnensumpf das entgaste Hydroformylierungsprodukt abgezogen wird, das der weiteren Aufarbeitung zugeführt werden kann, und wobei am Kolonnenkopf ein flüssiger oder gasförmiger Strom abgezogen wird, der im Wesentlichen aus nicht umgesetztem Propylen und Propan besteht.

Vorteilhaft wird die Entgasung des rohen Hydroformylierungsprodukts in einer Kolonne bei im Allgemeinen 4 bis 12 bar, vorzugsweise 6 bis 10 bar und besonders bevorzugt 6 bis 8 bar durchgeführt, wobei je nach angewandtem Druck, die Temperatur im Sumpf der Kolonne im Bereich von im Allgemeinen 120 bis 190°C, vorzugsweise 140 bis 170°C und besonders bevorzugt 140 bis 160°C gehalten wird. Als Trenneinbauten können in der Entgasungskolonne z.B. Packungen, Füllkörper oder Böden verwendet werden, vorzugsweise werden Packungen benutzt. Die Anzahl der theoretischen Böden der Entgasungskolonne liegt im Allgemeinen im Bereich von 8 bis 40, vorzugsweise im Bereich von 10 bis 25 und besonders bevorzugt im Bereich von 10 bis 20 Zweckmäßigerweise wird die Entgasungskolonne so betrieben, daß der Gehalt an Butyraldehyden im daraus erhaltenen Propylen- und Propan enthaltenden Strom 0,5 Gew.-%, vorzugsweise 0,1 Gew.-% und besonders bevorzugt 0,01 Gew.-% nicht übersteigt. Die genauen Betriebsbedingungen der Entgasungskolonne können entsprechend der Trennleistung der verwendeten Kolonne vom Fachmann anhand der bekannten Dampfdrucke und Verdampfungsgleichgewichte der im rohen Hydroformylierungsprodukt enthaltenen Komponenten nach herkömmlichen Berechnungsmethoden routinemäßig ermittelt werden.

Vorteilhaft können statt einer einzigen Entgasungskolonne zwei Entgasungskolonnen verwendet werden, wobei diese vorzugsweise bei unterschiedlichen Bedingungen betrieben werden. Die erste Entgasungskolonne wird bei dieser Ausgestaltung im allgemeinen bei einem niedrigeren Druck betrieben als die zweite Entgasungskolonne. Beispielsweise kann die erste Kolonne unter vorstehend angegebenen Druck- und Temperaturbedingungen betrieben und die zweite Kolonne bei einem Druck von im allgemeinen 6 bis 30 bar vorzugsweise 10 bis 26 bar und bei Temperaturen von im allgemeinen von 140 bis 200°C betrieben werden. Durch diese Verfahrensweise kann in der ersten Kolonne auf besonders schonende Weise entgast werden, wohingegen bei der Entgasung bei höherem Druck in der zweiten Kolonne eine bessere Abtrennung, beispielsweise von Butyraldehyden, erzielt wird.

Je nach Art des in der Gasphasenoxidation zu Acrolein und/oder Acrylsäure verwendeten Katalysators, kann es auch vorteilhaft sein, den Wassergehalt des aus der Entgasungskolonne gewonnenen, Propylen und Propan enthaltenden Stroms so einzustellen, daß er 50 ppm nicht übersteigt. Diese Maßnahme erweist sich zusätzlich dann als besonders vorteilhaft, wenn der Propen und Propan enthaltende Strom der Acrolein- und/oder Acrylsäureanlage in flüssiger Form zugeführt wird. Dies kann dadurch bewerkstelligt werden, daß man z.B. in der Rückflußleitung am Kopf der Entgasungskolonne einen Phasenabscheider installiert, in dem azeotrop mit Propylen und Propan abdestilliertes Wasser (gebildet durch Kondensation der Aldehyde zu hochsiedenden Nebenprodukten) vom als Rückfluß auf die Kolonne gegebenen Propylen-Propan-Gemisch abgetrennt wird. Alternativ kann auch das flüssige Propylen-Propan-Gemisch vor seiner Zuführung in die Gasphasenoxidation über einen Phasenabscheider oder, falls besonders niedrige Wassergehalte angestrebt werden, über ein Trocknungsmittel, wie Molekularsieb 4Å oder Molekularsieb 3Å, zwecks Abtrennung des Wassers geleitet werden. Der Wassergehalt eines gegebenenfalls gasförmigen Propen-Propan-Stroms kann gewünschtenfalls ebenfalls durch Adsorption des Wassers an einem Trocknungsmittel, z.B. Molekularsieb 4Å oder Molekularsieb 3Å, erniedrigt werden.

Mit den vorstehend beschriebenen Maßnahmen gelingt es, den Gehalt des der Gasphasenoxidation zu Acrolein und/oder Acrylsäure oder gegebenenfalls einer dieser Gasphasenoxidation vorgeschalteten oxidehydrierungsstufe, in der ein Teil des Propans einer oxidierten Dehydrierung zu Propylen unterzogen und somit der Propylen und Propan enthaltende Strom an Propylen angereichert wird, an Butyraldehyden und gewünschtenfalls an Wasser auf die vorstehend angegebenen Werte abzusenken. Phosphorhaltige Verbindungen (z.B. freier Ligand aus der Hydroformylierung) sind bei Anwendung der genannten Bedingungen in dem aus der Entgasungskolonne erhaltenen Propylen-Propan-Strom im Allgemeinen nicht nachweisbar.

Die Abtrennung des rohen Hydroformylierungsproduktes, das nicht umgesetztes Propylen und Propan gelöst enthält, vom Austrag aus der Reaktionszone kann auf verschiedene Weise erfolgen. Es kann zum Beispiel ein Flüssigaustragsverfahren zum Einsatz kommen, bei dem der im Wesentlichen, bis auf das im Überschuss zur Hydroformylierung eingesetzte Synthesegas, flüssige Austrag aus der Reaktionszone entspannt wird, wobei infolge der Druckerniedrigung der Austrag in eine Flüssigphase, die im Wesentlichen aus hochsiedenden Nebenprodukten, dem homogen gelösten Hydroformylierungskatalysator, einem Teil des Hydroformylierungsprodukts und gelöstem, nicht umgesetztem Propylen und Propan besteht, und eine Gasphase, die im Wesentlichen aus Hydroformylierungsprodukt, nicht umgesetztem Propylen und Propan sowie nicht umgesetztem Kohlenmonoxid und Wasserstoff sowie Inerten (z.B. N₂, CO₂, Methan) besteht, aufgetrennt wird. Die Flüssigphase kann, gegebenenfalls nach weiterer Abtrennung darin enthaltener Produktaldehyde, als Rückführstrom wieder in den Reaktor geleitet werden. Durch zumindest partielle Kondensation der Gasphase wird das rohe Hydroformylierungsprodukt erhalten. Die bei der Kondensation zurückbleibende Gasphase wird ganz oder teilweise in die Reaktionszone zurückgeführt.

Mit Vorteil kann die in der Entspannungsstufe primär anfallende Gas- und Flüssigphase nach dem in der WO 97/07086 beschriebenen Verfahren aufgearbeitet werden. Zu diesem Zweck wird die Flüssigphase erwärmt und in den oberen Bereich einer Kolonne eingeleitet, während die Gasphase in den Sumpf der Kolonne eingeleitet wird. Flüssigphase und Gasphase werden dadurch im Gegenstrom geführt. Um den gegenseitigen Kontakt der Phasen zu erhöhen, ist die Kolonne vorzugsweise mit Füllkörpern gefüllt. Durch den innigen Kontakt der Gasphase mit der Flüssigphase werden die in der Flüssigphase vorhandenen Restmengen an Hydroformylierungsprodukt, nicht umgesetztem Propylen und Propan in die Gasphase transferiert, so dass der die Kolonne am Kopf verlassende Gasstrom im Vergleich zu dem am unteren Ende der Kolonne eingeführten Gasstrom an Hydroformylierungsprodukt sowie nicht umgesetztem Propylen und Propan angereichert ist. Die weitere Aufarbeitung des die Kolonne verlassenden Gasstroms und der die Kolonne verlassenden Flüssigphase erfolgt wie oben beschrieben, z.B. in einer Entgasungskolonne.

Alternativ kann man nach dem sogenannten Kreisgasverfahren verfahren, bei dem aus dem Gasraum des Hydroformylierungsreaktors ein Gasstrom abgezogen wird. Dieser Gasstrom besteht im Wesentlichen aus Synthesegas, nicht umgesetztem Propylen und Propan, wobei nach Maßgabe des Dampfdrucks im Hydroformylierungsreaktor das bei der Hydroformylierungsreaktion gebildete Hydroformylierungsprodukt mitgeführt wird. Aus dem Gasstrom wird das mitgeführte rohe Hydroformylierungsprodukt z. B. durch Abkühlen auskondensiert, und der vom Flüssiganteil befreite Gasstrom wird zurück in den Hydroformylierungsreaktor geführt. Das im auskondensierten, rohen Hydroformylierungsprodukt gelöst enthaltene, nicht umgesetzte Propylen und Propan kann dann, wie beschrieben, z.B. in einer Entgasungskolonne freigesetzt werden.

Der im Wesentlichen aus nicht umgesetztem Propylen und Propan bestehende Strom enthält z. B. 40 bis 80 Gew.-%, vorzugsweise 60 bis 80 Gew.-% Propylen und 20 bis 60 Gew.-%, vorzugsweise 20 bis 40 Gew.-% Propan.

Lediglich beispielhaft wird nachstehend eine typische Zusammensetzung, einschließlich Verunreinigungen, eines nicht umgesetztes Propylen und Propan enthaltenden Stroms, wie er bei der Entgasung mittels zweier Entgasungskolonnen und anschließender Trocknung mittels Molekularsieb 3Å freigesetzt werden kann, angegeben:

| | |
|---|---|
| Propylen | 68 Gew.-% |
| Propan | 30 Gew.-% |
| Methan | 1,7 Gew.-% |
| Kohlenmonoxid | 0,28 Gew.-% |
| Kohlendioxid | 0,38 Gew.-% |
| Ethan | 0,20 Gew.-% |
| Wasserstoff | 350 Gew.-ppm |
| Butyraldehyde | 10 Gew.-ppm |
| Wasser | 5 Gew.-ppm. |

Der gasförmige, im Wesentlichen aus nicht umgesetztem Propylen und Propan bestehende Strom wird nach an sich bekannten Verfahren einer katalytischen Gasphasenoxidation zur Herstellung von Acrylsäure und/oder Acrolein unterworfen. Der Strom kann selbstverständlich mit propylenhaltigen Zuläufen aus anderen Quellen, z. B. einem Steamcracker, gemischt werden, um einen Zulauf zur Gasphasenoxidation zu bilden. In diesem Fall kann dann zum Beispiel ein Gemischpropenstrom der nachfolgenden Zusammensetzung für die Gasphasenoxidation zu Acrolein und/oder Acrylsäure resultieren und verwendet werden:

| | |
|---|---|
| Propylen | ≥ 94 Gew.-% |
| Propan | ≤ 6 Gew.-% |
| Methan und/oder Ethan | ≤ 4000 Gew.-ppm |
| C₄-Kohlenwasserstoffe | ≤ 5 Gew.-ppm |
| Acetylen | ≤ 1 Gew.-ppm |
| Wasser | ≤ 5 Gew.-ppm |
| Wasserstoff | ≤ 100 Gew.-ppm |
| Sauerstoff | ≤ 2 Gew.-ppm |
| Schwefelhaltige Verbindungen (berechnet als Schwefel) | ≤ 2 Gew.-ppm |
| Chlorhaltige Verbindungen (berechnet als Chlor) | ≤ 1 Gew.-ppm |
| CO₂ | ≤ 1000 Gew.-ppm |
| CO | ≤ 700 Gew.-ppm |
| Cyclopropan | ≤ 10 Gew.-ppm |
| Propadien und/oder Propin | ≤ 5 Gew.-ppm |
| Kohlenwasserstoffe > C₅ (Grünöle) | ≤ 10 Gew.-ppm |
| Carbonylgruppen-haltige Verbindungen (berechnet als Ni(CO₄) | ≤ 10 Gew.-ppm |
| Butyraldehyde | ≤ 2,5 Gew.-ppm |

P- und/oder As-haltige Verbindungen: nicht nachweisbar

Der gegebenenfalls mit einem inerten Verdünnungsgas gemischte Zulauf wird im Gemisch mit Sauerstoff bei erhöhten Temperaturen, üblicherweise 200 bis 450 °C, sowie gegebenenfalls erhöhtem Druck über einen heterogenen Katalysator, in der Regel übergangsmetallische, z. B. Molybdän, Vanadium, Wolfram und/oder Eisen enthaltende Mischoxidkatalysatoren, geleitet und dabei oxidativ in Acrylsäure und/oder Acrolein umgewandelt. Hierzu wird z. B. auf die DE-A-4405059, EP-A-0253409, EP-A-0092097 und DE-A-4431949 verwiesen. Die Umsetzung zu Acrylsäure kann einstufig oder zweistufig durchgeführt werden. Bei zweistufiger Reaktionsführung wird das Propylen in einer ersten Stufe zu Acrolein oxidiert und das Acrolein in einer zweiten Stufe zu Acrylsäure oxidiert. Als heterogene Katalysatoren sind in der ersten Stufe oxidische Mehrkomponenten-Katalysatoren auf der Basis der Oxide von Molybdän, Bismut und Eisen und in der zweiten Stufe entsprechende Katalysatoren auf der Basis der Oxide von Molybdän und Vanadium bevorzugt.

Das Propan kann in der Gasphasenoxidation als Verdünnungsgas und/oder Einsatzstoff dienen. Ein geeignetes Verfahren, bei dem Propan als Einsatzstoff direkt zu Acrylsäure umgesetzt wird, ist in der EP-B-0608838 beschrieben. Alternativ kann zur Erhöhung des Propylenanteils im bei der Hydroformylierung anfallenden Propylen/Propan-Strom dieser Strom vor seiner Verwendung als Einsatzstoff bei der Herstellung von Acrylsäure einer katalytischen Oxidehydrierung, wie z. B. in Catalysis Today, 24 (1995), 307-313 oder der US-A-5510558 beschrieben, einer homogenen oxidehydrierung oder einer katalytischen Dehydrierung, wie in der DE-A-19508558, EP-A-0293224 oder der EP-A-0117146 beschrieben, unterworfen werden.

Die Umsetzung von Propylen zu Acrylsäure und/oder Acrolein ist stark exotherm. Der Propylen/Propan-Strom wird daher in vorteilhafter Weise mit einem inerten Verdünnungsgas, z. B. Luftstickstoff, Kohlendioxid, Methan und/oder Wasserdampf verdünnt. Obwohl die Art der verwendeten Reaktoren an sich keiner Beschränkung unterliegt, werden zweckmäßigerweise Rohrbündelwärmetauscher verwendet, die mit dem Oxidationskatalysator gefüllt sind, da bei diesen der überwiegende Teil der bei der Reaktion freiwerdenden Wärme durch Wärmeleitung, Konvektion und Strahlung an die gekühlten Reaktorwände abgeführt werden kann.

Bei der ein- oder zweistufigen katalytischen Gasphasenoxidation zu Acrylsäure wird nicht reine Acrylsäure, sondern ein gasförmiges Gemisch erhalten, das neben der Acrylsäure als Nebenkompenenten im Wesentlichen nicht umgesetztes Acrolein und/oder Propylen, Propan, Wasserdampf, Kohlenmonoxid, Kohlendioxid, Stickstoff, Sauerstoff, Essigsäure, Propionsäure, Formaldehyd, weitere Aldehyde und Maleinsäureanhydrid enthalten kann.

Aus den erhaltenen Reaktionsgasen kann die Acrylsäure z. B. durch Gegenstromabsorption mit einem hochsiedenden inerten Lösungsmittel, z. B. Diphenylether, Diphenyl und/oder Dimethylphthalat und dergleichen abgetrennt werden, vergleiche DE 2136396 und DE-A-4308087 und anschließend z.B. destillativ isoliert werden.

Andererseits ist es möglich, das in der Gasphasenoxidation erhaltene Reaktionsprodukt einer Kondensation, insbesondere einer Partial- oder Totalkondensation, zu unterziehen, und aus der erhaltenen Lösung die Acrylsäure durch Kristallisation abzutrennen. Die Kondensation wird vorzugsweise in einer Kolonne durchgeführt. Hierbei wird die Kolonne mit trennwirksamen Einbauten, insbesondere mit Packungen, Füllkörpern Und/oder Böden, vorzugsweise Glockenböden, Siebböden, Ventilböden und/oder Dual-Flow-Böden, eingesetzt. Dabei werden die kondensierbaren Komponenten des gasförmigen Produktgemischs aus der Gasphasenoxidation fraktioniert durch Kühlung auskondensiert. Da das Gasgemisch in der Regel in Folge von Verunreinigungen und gegebenenfalls mitverwendeter Verdünnungsgase eine Schwersieder-, Mittelsieder- und Leichtsiederfraktion sowie nicht kondensierbare Komponenten enthält, können bei der Kolonne an den entsprechenden Stellen ein oder mehrere Seitenabzüge vorgesehen sein. Die Kondensation in der Kolonne ermöglicht somit bereits eine Auftrennung in die einzelnen Komponenten. Geeignete Kolonnen umfassen wenigstens eine Kühlvorrichtung, wofür sich alle gängigen Wärmeübertrager oder Wärmetauscher eignen, bei denen die bei der Kondensation gebildete wärme indirekt abgeführt wird. Bevorzugt sind Rohrbündelwärmetauscher, Plattenwärmetauscher und Luftkühler. Geeignete Kühlmedien sind z.B. Luft bzw. Kühlflüssigkeiten, wie insbesondere Wasser. Der in der Kolonne herrschende Druck hängt von der Menge an nicht kondensierbaren Komponenten ab und beträgt vorzugsweise 0,5 bis 5 bar Absolutdruck, insbesondere 0,8 bis 3 bar Absolutdruck. Die genauen Betriebsbedingungen für die Kolonne, wie die Temperaturund Druckführung, Schaltung und Anordnung der Kühlvorrichtung(en), können vom Fachmann im Rahmen üblicher Optimierungen in Abhängigkeit von der Gemischzusammensetzung ermittelt werden. In einer bevorzugten Ausführungsform wird das heiße Gasgemisch vor der Auskondensation direkt oder indirekt abgekühlt. Im Fall der direkten Kühlung wird als Kühlmedium vorzugsweise die bei der fraktionierten Kondensation anfallende Schwersiederfraktion verwendet. Alternativ kann ein nicht systemimmanentes Kühlmedium eingesetzt werden beispielsweise Diphenylether, Diphenyl und/oder Dimethylphthalat. Die Vorkühlung kann in den Sumpfbereich der Kolonne integriert oder getrennt von der Kolonne in einem eigenen Apparat, z. B. einem Gaskühler, einem Quench oder einem Flashtopf erfolgen.

Die bei der Kondensation erhaltene Lösung, die Acrylsäure enthält, kann anschließend kristallisiert werden. Die Kristallisation kann kontinuierlich oder diskontinuierlich, einstufig oder mehrstufig durchgeführt werden. Vorzugsweise erfolgt die Kristallisation einstufig. Die erhaltenen Kristalle von Acrylsäure werden anschließend von der Mutterlauge abgetrennt. Mit Vorteil wird die Mutterlauge zumindest teilweise in die Stufe der Kondensation des gasförmigen Produktgemischs zurückgeführt. Ein besonders bevorzugtes Verfahren zur Herstellung von Acrylsäure ist in der DE 197 40 252 beschrieben, auf die vollinhaltlich Bezug genommen wird.

Das die Gasphasenoxidation zu Acrolein und/oder Acrylsäure verlassende Abgas, das je nach angewandtem Oxidationsverfahren als wesentliche Wertkomponente nicht umgesetztes Propan enthält, kann z.B. als Cracker-Rohstoff oder als Rohstoff für eine Propanoxidehydrierungsanlage genutzt oder aber auch als Brennstoff zur Dampferzeugung oder Energiegewinnung verwendet werden.

Der mit Hilfe des erfindungsgemäßen Verfahrens erzielte Verbund von Butyraldehyd- und Acrylsäureproduktion ermöglicht eine optimale Ausnutzung des der Hydroformylierungsanlage zugeführten Propylenrohstoffs bei minimalem Aufwand und verbessert auf diese Weise die Wirtschaftlichkeit beider Verfahren. So war es bislang zur nahezu vollständigen Nutzung des der Hydroformylierungsanlage zugeführten Propylens - wollte man den im Abgas der Hydroformylierungsanlage enthaltenden Wertstoff Propylen nicht einfach, jedoch unrentabel als Brennstoff benutzen - erforderlich, das nicht umgesetzte Propylen vor seiner Rückführung in den Hydroformylierungsreaktor vom darin enthaltenen Propan in einer aufwendig zu betreibenden Propen/Propan-Trennanlage zu trennen. Auch die Verwendung des Propen und Propan enthaltenden Abgases der Hydroformylierungsanlage als Rohstoff für einen Steamcracker, falls ein solcher am betreffenden Standort überhaupt zur Verfügung steht, ist aus wirtschaftlicher Sicht weniger vorteilhaft als das erfindungsgemäße verfahren, da bezüglich des Propylenanteils in diesem Cracker-Rohstoff keine zusätzliche Wertschöpfung geschaffen wird. Eine andere Lösung zur möglichst vollständigen Nutzung des einer Hydroformylierungsanlage zugeführten Olefinrohstoffs wird in EP-A 188246 vorgeschlagen, nämlich die Nutzung des Propylen und Propan enthaltenden Abgases eines ersten Hydroformylierungsreaktors als Rohstoff in einem zweiten, vom ersten entkoppelten Hydroformylierungsreaktor. Doch auch diese Lösung verursacht eine signifikante Zusatzinvestition für den zweiten, entkoppelten Reaktor und natürlich auch zusätzliche Betriebskosten. Hingegen erlaubt das erfindungsgemäße Verfahren eine optimale Nutzung des Propylenrohstoffs der Hydroformylierungsanlage praktisch ohne nennenswerte zusätzliche Investitionen, da im Allgemeinen lediglich eine Versorgungsleitung zwischen Hydroformylierungs- und Acrylsäureanlage zu installieren ist und beim erfindungsgemäßen Betrieb der Entgasungskolonne zur Abtrennung eines für die Acrolein/Acrylsäure-Produktion geeigneten Propylen/Propan-Stroms praktisch keine nennenswerten, zusätzlichen Betriebskosten entstehen.

Der Erfolg des erfindungsgemäßen Verfahrens ist überraschend, da der Fachmann zu befürchten hatte, dass die Verwendung des Propylen und Propan enthaltenden Abgasstroms aus einer Hydroformylierungsanlage als Rohstoff zur Herstellung von Acrylsäure nachteilige Auswirkungen auf die Reinheit der daraus hergestellten Acrylsäure zur Folge hat und demzufolge deren Verwendungsund Vermarktungsmöglichkeiten einschränkt.

Die Erfindung wird durch das folgende Beispiel näher veranschaulicht.

### Beispiel

Einem Hydroformylierungsreaktor wurde ein Zulauf von 1 kg/h "Chemical Grade Propylen" mit einem Gehalt von 95 Gew.-% Propylen und 5 Gew.-% Propan sowie das für die Umsetzung notwendige Synthesegas zugeführt. Das gebildete Produkt, ein Gemisch aus n- und iso-Butyraldehyd, wurde zusammen mit nicht umgesetztem Propylen sowie dem zugeführten und dem gebildeten Propan mit Hilfe eines Kreisgasstroms aus dem Reaktor ausgetragen. Die kondensierbaren Anteile wurden in einem nachgeschalteten Kühler niedergeschlagen und einem darauffolgenden Abscheider gesammelt. Das Kondensat enthielt 78,3 Gew.-% Butyraldehyd, 14,3 Gew.-% Propylen und 7,4 Gew.-% Propan. Dieses Gemisch wurde einer Entgasungskolonne zugeführt (2,03 kg/h), wo es in einen C₃-freien Aldehydstrom (das Roh-Oxoprodukt) im Sumpf (1,6 kg/h) und ein Gemisch aus 66 Gew.-% Propylen und 34 Gew.-% Propan am Kopf (0,44 kg/h) aufgetrennt wurde.

Der Kopfabzug wurde mit dem Zulauf zur Synthesestufe einer Acrylsäureanlage (0,33 kg/h "Chemical Grade Propylen") vereinigt. Der Zulauf wies dementsprechend eine Zusammensetzung von 78,4 Gew.-% Propylen und 21,6 Gew.-% Propan auf.

Der Zulaufstrom wurde verdampft und zusammen mit Luft und Stickstoff durch ein Stahlrohr geleitet, das einen Oxidationskatalysator auf Mo-Basis 2, hergestellt gemäß Beispiel 1c), 1 von US-A 4298763, enthielt und auf einer Temperatur von etwa 340 °C gehalten wurde. Durch Umsetzung mit Sauerstoff erhielt man darin bei einem Propylenumsatz von 95,3 Mol-% eine Ausbeute an Acrolein von 87,4 und an Acrylsäure von 1 Mol-%.

In einem zweiten Experiment wurde das vorstehend erhaltene Acrolein-Produktgemisch ohne weitere Aufarbeitung durch ein zweites, gleich ausgestaltetes Stahlrohr geleitet, das mit einem Katalysator zur Oxidation von Acrolein zu Acrylsäure, hergestellt gemäß Beispiel 1 von US-A 4 259 211, gefüllt war. Vor dem Eintritt in das zweite Stahlrohr wurde dem Acrolein-Produktgemisch aus dem ersten Stahlrohr soviel Luft zugesetzt, dass am Austritt aus dem zweiten Stahlrohr der Sauerstoffgehalt des Reaktionsgemisches 3,2 vol.-% betrug. Bei einer Temperatur von 291 °C im zweiten Stahlrohr (Reaktor) wurde bei einem Acroleinumsatz von 99,3 % eine Acrylsäureausbeute von 95,2 %, bezogen auf das eingesetzte Acrolein, erzielt.

## Patentansprüche

1. Verfahren zur Herstellung von Hydroformylierungsprodukten des Propylens und von Acrylsäure und/oder Acrolein, bei dem man
a) in eine Reaktionszone einen Propylen-haltigen Zulauf sowie Kohlenmonoxid und Wasserstoff einspeist und in Gegenwart eines Hydroformylierungskatalysators zu Hydroformylierungsprodukten des Propens umsetzt,
b) vom Austrag aus der Reaktionszone einen im Wesentlichen. aus nicht umgesetztem Propylen und Propan bestehenden Strom abtrennt,
c) den im Wesentlichen aus Propylen und Propan bestehenden Strom in Gegenwart von molekularem Sauerstoff einer katalytischen Gasphasenoxidation zu Acrylsäure und/oder Acrolein unterwirft.

2. Verfahren nach Anspruch 1, bei dem der Propylen-haltige Zulauf 2 bis 40 Gew.-% Propan enthält.

3. Verfahren nach Anspruch 1 oder 2, bei dem man den im Wesentlichen aus nicht umgesetztem Propylen und Propan bestehenden Strom dadurch erhält, dass man vom Austrag aus der Reaktionszone zuerst ein rohes Hydroformylierungsprodukt abtrennt, welches nicht umgesetztes Propylen und Propan gelöst enthält, und das rohe Hydroformylierungsprodukt einem Entgasungsschritt unterzieht.

4. Verfahren nach Anspruch 3, bei dem der Austrag aus der Reaktionszone im Wesentlichen gasförmig ist und das rohe Hydroformylierungsprodukt durch partielle Kondensation des gasförmigen Austrags abgetrennt wird.

5. Verfahren nach Anspruch 3, bei dem der Austrag aus der Reaktionszone im Wesentlichen flüssig ist, der flüssige Austrag entspannt wird, wobei eine Auftrennung in eine Flüssigphase, die im Wesentlichen aus hochsiedenden Nebenprodukten, dem homogen gelösten Hydroformylierungskatalysator und geringen Mengen an Hydroformylierungsprodukt, geringen Mengen an nicht umgesetztem Propylen und geringen Mengen an Propan besteht, und eine Gasphase, die im Wesentlichen aus Hydroformylierungsprodukt, nicht umgesetztem Propylen und Propan sowie nicht umgesetztem Kohlenmonoxid und Wasserstoff besteht, erfolgt, und das rohe Hydroformylierungsprodukt durch zumindest partielle Kondensation der Gasphase erhalten wird.

6. Verfahren nach Anspruch 3, bei dem man für den Entgasungsschritt, dem das rohe Hydroformylierungsprodukt unterzogen wird, zwei Entgasungskolonnen einsetzt.

7. Verfahren nach einem der vorhergehenden Ansprüche, bei dem man als Hydroformylierungskatalysator einen phosphorhaltigen Rhodiumkatalysator verwendet.

8. Verfahren nach einem der vorhergehenden Ansprüche, bei dem der im Wesentlichen aus nicht umgesetztem Propylen und Propan bestehende Strom mit einem inerten Verdünnungsgas gemischt und in Gegenwart von Sauerstoff in wenigstens einer Oxidationszone mit einem heterogenen Katalysator in Kontakt gebracht wird.

9. Verfahren nach Anspruch 8, bei dem der im Wesentlichen aus nicht umgesetztem Propylen und Propan bestehende Strom in einer ersten Oxidationszone unter Erhalt eines Acrolein enthaltenden intermediären Produktgasgemischs mit einem ersten heterogenen Katalysator in Kontakt gebracht wird und das intermediäre Produktgasgemisch in einer zweiten Oxidationszone unter Erhalt eines Acrylsäure enthaltenden Produktgasgemischs mit einem zweiten heterogenen Katalysator in Kontakt gebracht wird.

10. Verfahren nach Anspruch 1, **dadurch gekennzeichnet, daß** man den im Wesentlichen aus nicht umgesetztem Propylen und Propan bestehenden Strom vor dessen katalytischer Gasphasenoxidation zu Acrylsäure und/oder Acrolein zur Erhöhung seines Propylenanteils einer Oxidehydrierung unterzieht.

## Claims

1. A process for preparing hydroformylation products of propylene and for preparing acrylic acid and/or acrolein, which comprises
a) feeding a propylene-containing feed and also carbon monoxide and hydrogen into a reaction zone and reacting this mixture in the presence of a hydroformylation catalyst to form hydroformylation products of propene,
b) separating a stream consisting essentially of unreacted propylene and propane from the output from the reaction zone,
c) subjecting the stream consisting essentially of propylene and propane to a catalytic gas-phase oxidation in the presence of molecular oxygen to form acrylic acid and/or acrolein.

2. A process as claimed in claim 1, wherein the propylene-containing feed contains from 2 to 40% by weight of propane.

3. A process as claimed in claim 1 or 2, wherein the stream consisting essentially of unreacted propylene and propane is obtained by firstly separating a crude hydroformylation product in which unreacted propylene and propane are present in dissolved form from the output from the reaction zone and subjecting the crude hydroformylation product to a degassing step.

4. A process as claimed in claim 3, wherein the output from the reaction zone is essentially gaseous and the crude hydroformylation product is separated off by partial condensation of the gaseous output.

5. A process as claimed in claim 3, wherein the output from the reaction zone is essentially liquid, the liquid output is depressurized, resulting in separation into a liquid phase consisting essentially of high-boiling by-products, the homogeneously dissolved hydroformylation catalyst and small amounts of hydroformylation product, small amounts of unreacted propylene and small amounts of propane and a gas phase consisting essentially of hydroformylation product, unreacted propylene and propane and unreacted carbon monoxide and hydrogen, and the crude hydroformylation product is obtained by at least partial condensation of the gas phase.

6. A process as claimed in claim 3, wherein two degassing columns are used for the degassing step to which the crude hydroformylation product is subjected.

7. A process as claimed in any of the preceding claims, wherein the hydroformylation catalyst used is a phosphorus-containing rhodium catalyst.

8. A process as claimed in any of the preceding claims, wherein the stream consisting essentially of unreacted propylene and propane is mixed with an inert diluent gas and brought into contact with a heterogeneous catalyst in the presence of oxygen in at least one oxidation zone.

9. A process as claimed in claim 8, wherein the stream consisting essentially of unreacted propylene and propane is brought into contact with a first heterogeneous catalyst in a first oxidation zone to give an acrolein-containing intermediate product gas mixture and the intermediate product gas mixture is brought into contact with a second heterogeneous catalyst in a second oxidation zone to give a product gas mixture comprising acrylic acid.

10. A process as claimed in claim 1, wherein the stream consisting essentially of unreacted propylene and propane is subjected to an oxydehydrogenation to increase its propylene content before it is subjected to catalytic gas-phase oxidation to form acrylic acid and/or acrolein.

## Revendications

1. Procédé de préparation de produits d'hydroformylation du propylène et d'acide acrylique et/ou d'acroléine, dans lequel on
a) introduit, dans une zone de réaction, une alimentation contenant du propylène ainsi que du monoxyde de carbone et de l'hydrogène et on la fait réagir, en présence d'un catalyseur d'hydroformylation, afin de former des produits d'hydroformylation du propène,
b) on sépare du produit issu de la zone de réaction un courant essentiellement composé de propylène n'ayant pas réagi et de propane,
c) on soumet le courant essentiellement composé de propylène et de propane à une oxydation catalytique en phase gazeuse, en présence d'oxygène moléculaire, afin de former de l'acide acrylique ou de l'acroléine.

2. Procédé selon la revendication 1, dans lequel l'alimentation contenant du propylène contient 2% à 40% de propane.

3. Procédé selon la revendication 1 ou 2, dans lequel on obtient le courant essentiellement composé de propylène n'ayant pas réagi et de propane en séparant du produit issu de la zone de réaction tout d'abord un produit d'hydroformylation brut, lequel contient le propylène n'ayant pas réagi et le propane sous forme dissoute, et en soumettant le produit d'hydroformylation brut à une étape de dégazage.

4. Procédé selon la revendication 3, dans lequel le produit issu de la zone de réaction est essentiellement gazeux et dans lequel on sépare le produit d'hydroformylation brut au moyen d'une condensation partielle du produit gazeux.

5. Procédé selon la revendication 3, dans lequel le produit issu de la zone de réaction est essentiellement liquide, dans lequel on détend le produit liquide, dans lequel on réalise une séparation en une phase liquide, qui se compose essentiellement de sous-produits à haut point d'ébullition, du catalyseur d'hydroformylation dissous de manière homogène et de faibles quantités de produit d'hydroformylation, de faibles quantités de propylène n'ayant pas réagi et de faibles quantités de propane, et en une phase gazeuse, qui se compose essentiellement du produit d'hydroformylation, de propylène n'ayant pas réagi et de propane ainsi que de monoxyde de carbone n'ayant pas réagi et d'hydrogène, et dans lequel on obtient le produit d'hydroformylation brut au moyen d'une condensation au moins partielle de la phase gazeuse.

6. Procédé selon la revendication 3, dans lequel on utilise, pour l'étape de dégazage à laquelle est soumis le produit d'hydroformylation brut, deux colonnes de dégazage.

7. Procédé selon l'une quelconque des revendications précédentes, dans lequel on utilise, en tant que catalyseur d'hydroformylation, un catalyseur de rhodium contenant du phosphore.

8. Procédé selon l'une quelconque des revendications précédentes, dans lequel on mélange le courant essentiellement composé de propylène n'ayant pas réagi et de propane avec un gaz diluant inerte et on le met en contact, en présence d'oxygène, dans au moins une zone d'oxydation, avec un catalyseur hétérogène.

9. Procédé selon la revendication 8, dans lequel on met en contact, dans une première zone d'oxydation, afin d'obtenir un mélange de produits gazeux intermédiaires contenant de l'acroléine, le courant essentiellement composé de propylène n'ayant pas réagi et de propane avec un premier catalyseur hétérogène et dans lequel on met en contact, dans une seconde zone d'oxydation, afin d'obtenir un mélange de produits gazeux contenant de l'acide acrylique, le mélange de produits gazeux intermédiaires avec un second catalyseur hétérogène.

10. Procédé selon la revendication 1, **caractérise en ce que** l'on soumet le courant essentiellement composé de propylène n'ayant pas réagi et de propane, avant son oxydation catalytique en phase gazeuse destinée à former de l'acide acrylique et/ou de l'acroléine, en vue d'élever sa teneur en propylène, à une oxydéshydrogénation.
